Europäisches Patentamt

(19) European Patent Office

Office européen des brevets

(11) Publication number: **0 135 135**
**A2**

# (12) EUROPEAN PATENT APPLICATION

(21) Application number: **84109626.6**

(22) Date of filing: **13.08.84**

(51) Int. Cl.⁴: **G 01 N 33/00**

(30) Priority: **15.08.83 US 523478**

(43) Date of publication of application:
**27.03.85 Bulletin 85/13**

(84) Designated Contracting States:
**DE GB IT**

(71) Applicant: **HONEYWELL INC.**
**Honeywell Plaza**
**Minneapolis Minnesota 55408(US)**

(72) Inventor: **Castleman, Bruce W.**
**6261 43rd Terrace North**
**Kenneth Florida 33709(US)**

(74) Representative: **Rentzsch, Heinz et al,**
**Honeywell Europe S.A. Holding KG Patent- und**
**Lizenzabteilung Kaiserleistrasse 55**
**D-6050 Offenbach am Main(DE)**

(54) **Method and apparatus for trace gas detection.**

(57) In the detection of trace components in a gas, by a process involving the ionization of said components, intermittently adding to the gas a reactant gas is provided which converts some of the components to more readily ionizable forms.

EP 0 135 135 A2

Croydon Printing Company Ltd.

0135135

August 10, 1984

A3410378 EP

HR/ep

HONEYWELL INC.

Honeywell Plaza

Minneapolis, Minn. USA

## METHOD AND APPARATUS FOR TRACE GAS DETECTION

This invention relates to the field of detection of ions, and more particularly to apparatus and procedures for detecting the presence of gaseous impurities or vapors in low concentration in a carrier gas including air.

The detection and identification of trace components of gases and vapors in the atmosphere requires highly sensitive and specific techniques. Examples of such techniques are mass spectroscopy, ion mobility spectroscopy, and ion diffusion techniques. These techniques require that the trace components be ionized or form ion clusters in order to be detected. One apparatus for use in these circumstances is an ionization detection cell shown in U.S. Patent 3,835,328.

It has been found that certain trace components are not easily ionized at atmospheric pressure, particularly in the presence of water vapor, and the sensitivity of the instrument for these substances is low. This can, to some extent, be corrected by a procedure which converts the recalcitrant components to forms, such as oxides, which are more easily ionized, and this can be done by judicious admixture of reactant gases such as ozone. This procedure has the disadvantage, however, that when concentrations of reactant gas are used sufficient to accomplish the desired results with particular trace components, the reactant gas has the effect of partially decomposing other trace components, so that the sensitivity of the apparatus to those other components is actually reduced.

It is the main object of the invention to improve the sensitivity of a ionizing gas detector for particular trace components without impairing its response to other components. To achieve this, the present invention provides the methods as characterized in claims 1 to 3. In a preferred embodiment ozone is intermittently admixed with a sample gas so that over a time period, the sensitivity of the instrument to various components is modulated, thereby alternately obtaining optimum detection of both types of components.

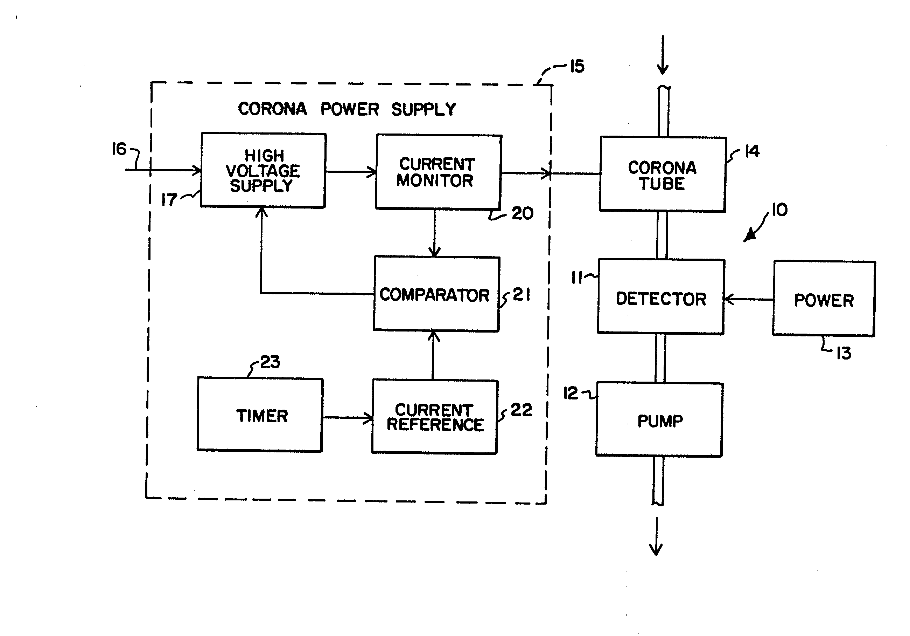

Various advantages and features of novelty which characterize the invention are pointed out with particularity in the subclaims. For a better understanding of the invention, its advantages, and objects attained by its use, reference should be had to the drawing in which there is illustrated and described a preferred embodiment of the invention.

Apparatus 10 according to the invention is shown to comprise a detector 11 through which the sample gas is drawn by a pump 12. Detector 11 may operate on the basis of mass spectroscopy, gas chromatography, ion mobility spectroscopy, or ion diffusion techniques, and includes apparatus for ionizing the trace components, from a suitable power source 13.

The sample gas is drawn to detector 11 through a source of reactant gas, shown as a corona tube 14 which converts some of the oxygen in the gas to ozone, its reactant form. It is not the function of tube 14 to replace the ionization function in detector 11, but simply to supply ozone for combining with the trace gas components to produce substances which are more readily ionizable in the presence of water vapor, such as sulfoxides or sulfones.

Corona tube 14 is energized from a power supply 15 which will now be described. Energy from a source 16 feeds a high voltage supply 17 which energizes tube 14 through a current monitor 20. A comparator 21 is fed from monitor 20 and a current reference 22 which is subject to a timer 23. By this arrangement, corona tube 14 is supplied with actuating voltage for first periods which alternate with second periods of voltage insufficient to cause ozone generation. The first and second periods are preferably of equal duration, although their lengths may be varied as desired.

Since pump 12 operates continuously, detector 11 is supplied with sample gas which for first intervals includes ozone and for second periods does not include ozone. During the first periods the sensitivity of detector 11 to trace gases such as 2-chloroethylsulfide is increased, while its sensitivity to gases such as dimethylmethylphosphonate is reduced. During the second periods, the sensitivity of the detector to dimethylmethylphosphonate is restored, while its sensitivity to 2-chloroethysulfide is reduced. Over a sufficient interval, detector 11 thus operates at good sensitivity for trace components of both kinds.

From the above it will be evident that the invention comprises an apparatus and a procedure for enhancing the sensitivity, of a detector based on ionization, to trace gases by intermittent admixture of reactant gas such as ozone to intermittently increase the ionizability of certain trace compontents.

Claims:

1. A method of detecting trace components in a gas which includes the step of ionizing said components, c h a r a c t e r i z e d   b y   the step of intermittently oxidizing the trace components into forms which are more easily ionizable.

2. A method of detecting trace components in a gas which includes the step of ionizing said components, c h a r a c t e r i z e d   b y   the step of intermittently mixing the gas to be tested with a reactant gas to convert the trace components into more easily ionizable forms.

3. The method according to claim 2, c h a r a c t e r - i z e d   i n   t h a t   said reactant gas is ozone.

4. Apparatus for detecting trace components in accordance with the method of claim 1, 2 or 3, c h a r a c - t e r i z e d   b y   an ionizing detector (11), a pump (12) for causing flow of a sample of gas to said detector, and a device (14) for intermittently converting said trace components, before reaching the detector (11), to more easily ionizable forms.

5. Apparatus according to claim 4, c h a r a c t e r - i z e d   i n   t h a t   said device (14) is a corona tube for converting part of the oxygen contained in the sample of gas into ozone.

6. Apparatus according to claim 4 or 5, c h a r a c - t e r i z e d   b y   a power supply unit (15) for said device (14) for intermittently energizing said device.

7. Apparatus according to claim 6, c h a r a c t e r - i z e d   i n   t h a t   the power supply unit (15) comprises a timer (23) for determining the time intervals during which the device (14) is alternately energized.

CORONA POWER SUPPLY

| HIGH VOLTAGE SUPPLY |
| CURRENT MONITOR |
| CORONA TUBE |
| COMPARATOR |
| DETECTOR |
| POWER |
| TIMER |
| CURRENT REFERENCE |
| PUMP |

0135135